# EUROPEAN PATENT APPLICATION

(11) **EP 4 344 547 A1**
(43) Date of publication of application: **03.04.2024**
(21) Application number: 23825351.2
(22) Date of filing: 04.07.2023
(51) Int. Cl.: A01N 63/20, A01P 3/00, A61K 31/70, A61K 35/74, A61K 35/744, A61P 31/10, C09D 5/14, A23L 33/10, A23K 10/16

(54) **CANDIDA ANTI-FUNGAL COMPOSITION CONTAINING LIPOTEICHOIC ACID**

(30) Priority: 01.08.2022 KR 20220095417
(71) Applicant: Seoul National University R & DB Foundation, Seoul 08826 (KR)
(72) Inventor: HAN, Seung Hyun, Yongin-si Gyeonggi-do 17125 (KR); PARK, Dong Hyun, Seoul 06262 (KR); LEE, Dongwook, Seoul 07650 (KR)
(74) Representative: Dehns
(86) International application number: PCT/KR2023/009382
(87) International publication number: WO 2024/029756

(57) **Abstract**

The present invention relates to an antifungal composition using lipoteichoic acid derived from various Gram-positive bacteria to inhibit the adhesion and aggregation of *Candida* fungi, a pharmaceutical composition for preventing and treating candidiasis infection and the use thereof. Using this approach, since the effect of inhibiting adhesion and cohesion, which are the representative virulence factors of *Candida* fungal infection and candidiasis, has been confirmed, it can be used for antifungal use against *Candida* fungi and for prevention or treatment related to candidiasis infection.

## Description

### [Technical Field]

The present invention relates to an antifungal composition using lipoteichoic acid derived from various Gram-positive bacteria to inhibit the attachment and aggregation of *Candida* fungi, a pharmaceutical composition for preventing and treating candidiasis infection and the use thereof.

### [Background Art]

*Candida* fungus is a higher organism fungus that is different from bacteria known to form existing biofilms, and it has the characteristic of being a eukaryotic cell, unlike bacteria, which are prokaryotic cells. *Candida* fungi grow in the form of yeast, pseudohyphae and hyphae depending on the environment, and this free cell shape conversion characteristic of *Candida* fungi causes infiltration into human tissues and facilitates evasion from phagocytic cells and dispersal in the bloodstream. As a result, it forms biofilms with strong adhesion to human tissues, bloodstream, or medical devices such as catheters that are artificially inserted into the human body. The surface adhesion of *Candida* fungi plays an important role in diseases caused by infection.

Candidiasis is caused by *Candida* fungi, which is a type of fungus. It is an infectious disease that occurs by infecting various parts of the body and causes symptoms such as thrush, vulvovaginitis and vaginitis. Among *Candida* fungi, *Candida albicans* is a common fungus that resides in the mucosa and stomach of healthy people, but it is also an opportunistic pathogen that causes infection in patients with impaired immune function or severe disease. In addition, Candidal vaginitis, which is a common vaginitis caused by *Candida albicans,* is a disease with a high frequency of occurrence, with an average of 7.5 out of 10 women getting the same at least once in their lifetime, and it can also cause various complications.

The biofilm of *Candida* fungi is formed through the strong adhesion and cohesion of fungi, and it has resistance to antifungal agents that is approximately 2,000 times higher than that of yeast-type cells. As a eukaryote, since it has common characteristics with human cells, it is difficult to develop drugs that are specific for fungal removal, and severe side effects occur in some patients, and thus, there is an urgent need to develop antifungal drugs with high therapeutic efficacy and low toxicity.

Lipoteichoic acid is a substance derived from Gram-positive bacteria and is one of the main components of the cell wall, and it plays a role in the growth and adhesion of bacteria and the activation of the host's immune system.

Korean Patent Application Laid-Open No. 10-2019-0017168 discloses a pharmaceutical composition of lipoteichoic acid for skin regeneration and wound healing, but does not disclose information about suppressing candidiasis.

Under this background, the inventors of the present invention have made extensive research efforts to develop a composition that can more effectively inhibit candidiasis, and as a result, the present invention was completed by confirming that lipoteichoic acid, which is isolated from Gram-positive bacteria, reduces the surface adhesion of *Candida* fungi.

### [Disclosure]

### [Technical Problem]

Therefore, an object of the present invention is to provide a *Candida* antifungal composition, including i) lipoteichoic acid or a pharmaceutically acceptable salt thereof; or ii) a cell wall extract including lipoteichoic acid.

Another object of the present invention is to provide a pharmaceutical composition for preventing or treating candidiasis infection, including the *Candida* antifungal composition.

Still another object of the present invention is to provide a coating agent, health functional food, livestock feed additive and foodstuff wash, including the *Candida* antifungal composition.

However, the technical problems to be achieved by the present invention are not limited to the problems mentioned above, and other problems that are not mentioned will be clearly understood by those skilled in the art from the description below.

### [Technical Solution]

Accordingly, the inventors of the present invention isolated and purified lipoteichoic acid with high purity from *Lactobacillus,* which is known as Gram-positive bacteria and a representative probiotic, and performed various efficacy evaluations on fungal cells. As a result, even at low dosages, the effects of lipoteichoic acid alone were confirmed in vitro and ex vivo experimental models for their ability to block adhesion and cohesion, which are characteristic virulence factors of Candida fungal infection and candidiasis. In addition, the present invention was completed by elucidating the mechanism of action that the inhibitory effect occurs when lipoteichoic acid interacts with Hsp70.

Accordingly, the present invention provides a *Candida* antifungal composition, including i) lipoteichoic acid or a pharmaceutically acceptable salt thereof; or ii) a cell wall extract including lipoteichoic acid.

As used herein, the term "antifungal" refers to the ability to resist microorganisms and the ability to inhibit the growth or proliferation of microorganisms, and it refers to all mechanisms to protect against the action of microorganisms such as bacteria, mold, yeast and the like, and in the present invention, it may refer to the ability to interfere with the action of *Candida* fungi.

Lipoteichoic acid or a cell wall extract including lipoteichoic acid included in the antifungal composition of the present invention may be derived from Gram-positive bacteria.

Preferably, the Gram-positive bacteria may be any one selected from the group consisting of strains including *Lactobacillus reuteri, Lactobacillus acidophilus, Lactobacillus rhamnosus* GG, *Lactobacillus plantarum, Lactobacillus casei, Lactobacillus paracasei, Lactobacillus fermentum, Lactobacillus delbrueckii subsp. bulgaricus, Lactobacillus helveticus, Lactobacillus fermentum, Lactobacillus salilvarius, Lactococcus lactis, Enterococcus faecalis, Enterococcus faecium, Streptococcus thermophilus, Bifidobacterium bifidum, Bifidobacterium breve, Bifidobacterium longum, Bifidobacterium animalis subsp. Lactis* and *Bacillus subtilis,* and subspecies of these strains, and the lipoteichoic acid or cell wall extract including lipoteichoic acid may be derived from the bacteria, but the present invention is not limited thereto, and any Gram-positive bacteria may be used as an available strain.

In the present invention, the cell wall extract may be extracted by using water, C₁ to C₄ lower alcohol or a mixture thereof as a solvent. Thereafter, it may be separated and purified by applying sequential hydrophobic interaction chromatography and anion exchange chromatography.

The solvent used for the extraction in the present invention may be used without limitation as long as it is a substance that can be commonly used as an extraction solvent, and preferably, at least any one selected from the group consisting of methanol, ethanol, propanol, isopropanol, butanol and a combination thereof may be used, and more preferably, butanol may be used.

In the *Candida* antifungal composition, *Candida* may be fungi selected from the group consisting of *Candida albicans, Candida tropicalis, Candida pseudotropicalis, Candida krusei, Candida glabrata* and a mixture thereof, but the present invention is not limited thereto.

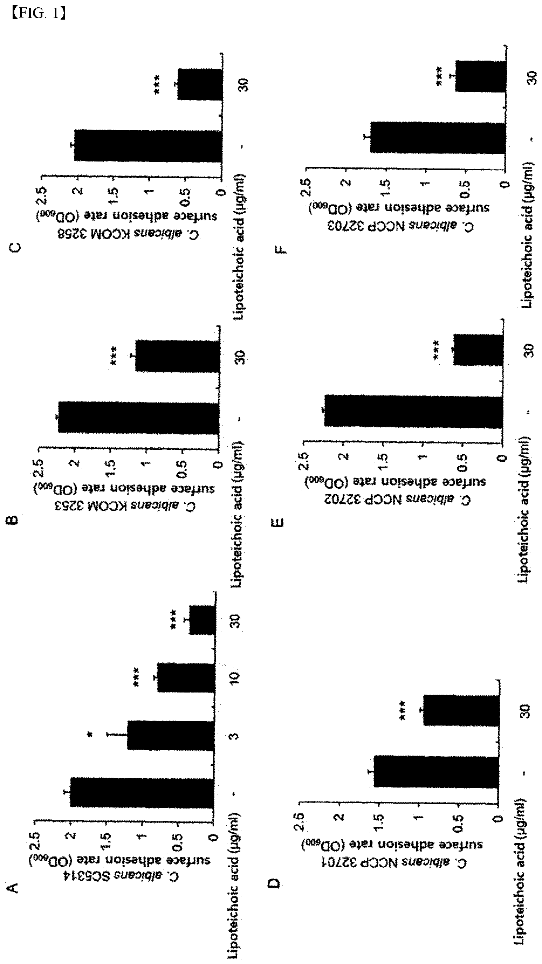

In the present invention, the composition may reduce the surface adhesion of *Candida* fungi. In a specific exemplary embodiment, as shown in FIGS. 1 to 3, when it is treated with the composition including lipoteichoic acid compared to the control group that was not treated with the composition, it was confirmed that the surface adhesion rate of *Candida* fungi was significantly weakened.

In addition, many antifungal drugs are currently being used to treat candidiasis caused by the *Candida* fungus. However, *Candida* fungi, which are fungi, are similar in many ways to the cells of higher animals, including humans, and thus, antifungal drugs that affect these fungi are also effective in the host, and as a result, since strong antifungal drugs have the potential to be toxic to the host, there have been limitations to the widespread use of antifungal drugs. In addition, with the recent increase in fungi (especially *Candida auris*) that have acquired or have intrinsic resistance to antifungal drugs, resistance to antifungal drugs has emerged as an important problem in the treatment of fungal infections (Protein J 2013, 32:15-26). Therefore, there was a need to develop an antifungal agent that is effective in treating fungal infections, has fewer side effects and less resistance, and can be used for a long period of time.

Additionally, the present invention provides a pharmaceutical composition for preventing or treating candidiasis infection, including the composition.

As used herein, the term "candidiasis" refers to mycosis caused by *Candida.* For example, candidiasis caused by *Candida albicans* was first isolated from a thrush patient and causes vaginitis in women and diaper rash in infants.

As used herein, the term "prevention" refers to any action that suppresses or delays the onset of candidiasis by administering the pharmaceutical composition of the present invention, and the term "treatment" refers to any action that alleviates or ameliorates the symptoms of candidiasis that are already caused by administration of the pharmaceutical composition of the present invention.

As used herein, the term "infection caused by *Candida* fungi" may be an infection that can be suppressed by the antifungal effect of the antifungal composition of the present invention, which includes infections that are treated with existing disinfectants and antibiotics and cases where infections may be caused when the disease is diagnosed.

The pharmaceutical composition of the present invention may additionally include appropriate carriers, excipients or diluents according to conventional methods. Examples of the carriers, excipients and diluents that may be included in pharmaceutical compositions including the compound include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil, but the present invention is not limited thereto.

The pharmaceutical composition of the present invention may be formulated as any one dosage form selected from the group consisting of powders, pills, granules, capsules, suspensions, oral solutions, emulsions, syrups, sterilized aqueous solutions, non-aqueous solutions, suspensions, freeze-dried preparations and suppositories by any method selected. When it is formulated, it may be prepared by using diluents or excipients such as commonly used fillers, extenders, binders, wetting agents, disintegrants and surfactants. Suitable agents known in the art are preferably those disclosed in the literature (Remington's Pharmaceutical Science, Mack Publishing Company, Easton PA).

The pharmaceutical composition of the present invention is administered in a pharmaceutically effective amount. As used herein, the term "pharmaceutically effective amount" refers to an amount sufficient to exhibit an antifungal effect against *Candida* fungi and an amount sufficient not to cause side effects or a serious or excessive immune response, and the effective dose level will depend on the disorder being treated, the severity of the disorder, the activity of the particular compound, the route of administration, the duration of treatment, the drugs used in combination or simultaneously with the composition including lipoteichoic acid, the subject's age, weight, gender and eating habits, and a variety of factors, including general health, and factors known in the pharmaceutical and medical fields. Various general considerations in determining a "therapeutically effective amount" are known to those skilled in the art and are described, for example, in Gilman et al., eds., Goodman And Gilman's: The Pharmacological Bases of Therapeutics, 8th ed., Pergamon Press, 1990] and [Remington's Pharmaceutical Sciences, 17th ed., Mack Publishing Co., Easton, Pa., 1990].

As used herein, the term "administration" refers to introducing a predetermined substance into a patient by any appropriate method, and it is formulated for human or veterinary use and administered through various routes. The composition of the present invention may be administered by parenteral routes, such as intravascular, intravenous, intraarterial, intramuscular or subcutaneous, may also be administered by oral, nasal, rectal, transdermal or inhalation routes via aerosol, and may be administered as a bolus or infused slowly.

The pharmaceutical composition of the present invention may be administered as an individual therapeutic agent or in combination with other therapeutic agents, and may be administered sequentially or simultaneously with conventional therapeutic agents. In addition, it may be administered single or multiple times. Considering all of the above factors, it is important to administer an amount that can achieve the maximum effect with the minimum amount without side effects, and it may be easily determined by a person skilled in the art.

The preferred dosage of the pharmaceutical composition of the present invention varies depending on the patient's condition and weight, degree of disease, drug form, administration route and period, but it may be appropriately selected by a person skilled in the art. However, for a desirable effect, it is recommended to administer 0.01 mg/kg to 10 g/kg per day, and preferably, 1 mg/kg to 1 g/kg. Administration may be administered once a day, or may be administered in several divided doses. Accordingly, the above dosage does not limit the scope of the present invention in any way.

The pharmaceutical composition of the present invention may include at least one known active ingredient that has effects in preventing and treating candidiasis along with the composition including lipoteichoic acid.

The pharmaceutical composition of the present invention may be used in prescription drugs, hygiene products and the like to perform an antifungal function against *Candida* fungi.

For the hygiene products, when it is added as an active ingredient to hygiene products such as oral cleansing products such as gargles and Listerine, toothpaste, oral hygiene products, soap, hand soap, vaginal cleanser additives, skin cleansing compositions such as body cleansers, kitchen utensil cleaning compositions for cleaning dishes, sinks, cutting boards and the like, and cosmetic compositions such as essences, creams and the like, it may perform an antifungal function against *Candida* fungi in addition to the original function of each hygiene product.

In the present invention, the candidiasis for which the pharmaceutical composition is effective may be at least any one selected from the group consisting of vulvovaginitis, vaginitis, esophagitis, paronychia, oropharyngitis, digestive candidiasis, mucosal candidiasis, cutaneous candidiasis, Candida fungemia, Candida endophthalmitis, Candida peritonitis, Candida osteoarthritis, Candida spondylitis and hepatosplenic candidiasis, but the present invention is not limited thereto.

For example, the term "vaginal candidiasis" refers to a specific type of vaginal mycosis caused by fungi of the genus *Candida* selected from the group consisting of *Candida albicans, Candida tropicalis, Candida pseudotropicalis, Candida krusei, Candida glabrata* and a mixture thereof. Vaginal candidiasis is also called candidal vaginitis.

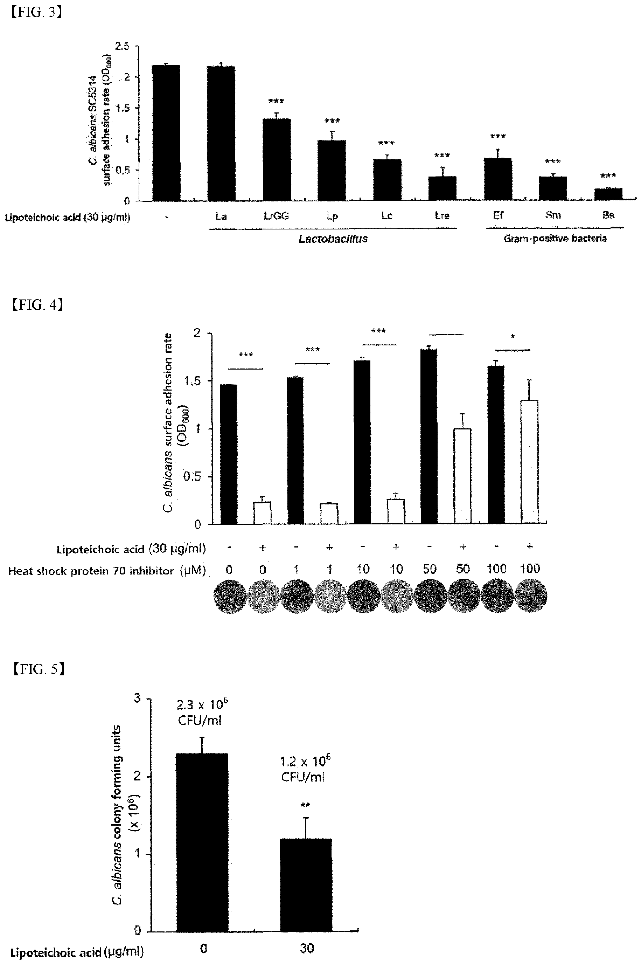

The pharmaceutical composition of the present invention interacts with Hsp70, which is a surface protein of *Candida* fungi, to reduce adhesion, thereby inhibiting biofilm formation and inhibiting fungal infection (refer to FIGS. 3 and 4).

Additionally, the present invention provides a method for preventing or treating candidiasis, including the step of administering the pharmaceutical composition to a subject who is expected to develop candidiasis or has developed candidiasis.

As used herein, the term "subject" refers to a living organism that can develop candidiasis, and preferably, it may be a higher vertebrate that can show the symptoms of candidiasis, and more preferably, it may be livestock such as cattle, pigs, sheep, goats and dogs in which candidiasis may occur, and humans that can be infected therefrom.

When the pharmaceutical composition of the present invention is administered to the subject, it may exhibit the effect of preventing or treating candidiasis, by improving the subject's immunity against strains that cause candidiasis. The subject of administration of this pharmaceutical composition may primarily be livestock such as cattle, pigs, sheep, goats and dogs, but the present invention is not limited thereto, and it may also be used for the prevention or treatment of humans who may be infected with candidiasis by the livestock.

Additionally, the present invention provides a coating agent, including the composition.

As used herein, the term "antifungal coating agent" is a coating agent to prevent surface adhesion and biofilm formation formed by fungi. In order to remove the biofilm formed after the attachment of microorganisms, since a high concentration of disinfectant or antibiotic is administered because it has strong resistance to microbial inhibitors, it may cause serious damage to the surrounding area, and thus, it may be used to prevent surface adhesion and biofilm formation in advance.

In the present invention, locations where biofilms can be formed may be moist areas of mucous membranes and skin, including the skin, oral cavity, digestive tract and vagina. As an example, areas that cause stomatitis include the tongue, inside the cheek, the palate including the oral cavity, oral lining, throat, esophagus and genital area, including the vulva, vagina and penis, and it may also include moist skin, fingernails or toenails, the groin, armpits, between fingers and toes, skin folds under the breasts and skin folds on the abdomen, but the present invention is not limited thereto.

Additionally, examples thereof include Sutures, extraction sites, arteriovenous sites, scleral buckle sites, contact lenses, IUDs, tracheal tubes, Hickman catheters, central venous catheters, artificial heart valves, valve grafts, orthopedic devices, penile prostheses and the like, and other examples are described in a research article by Costerton J et al. and Costerton J and Steward (2001 Battling Biofilms, Scientific American pp 75-81), and the above research article is incorporated herein by reference. Other locations where biofilms can be formed include cavities, which can progress to gum disease and cavities, contact lenses, which can progress to eye infections, ears, which can be chronically infected, and lungs in which pneumonia may occur.

Therefore, the coating agent may be used in medical devices, medical materials, medical implants, building interior materials, household goods, industrial products, electronic products, clothing and artificial organs, but the present invention is not limited thereto, and it may be used in places where there is a risk of contamination or infection with *Candida* fungi.

Among these, the medical devices refer to all devices that assist medical practices for performing surgery, treatment, prevention and the like, and examples thereof may include vascular stents, artificial blood vessels, artificial valves, blood collection devices, hemodialysis equipment and devices, and the aforementioned catheters and the like, but the present invention is not limited thereto, and they refer to devices that may be contaminated or infected by *Candida* fungi.

Additionally, the present invention provides a health functional food, including the composition.

As used herein, the term "health functional food" refers to food manufactured and processed by using raw materials or ingredients with functionality that is useful to the human body in accordance with Act No. 6727 on Functional Food, and the term "functionality" refers to intake for the purpose of controlling nutrients for the structure and function of the human body or obtaining useful effects for health purposes such as physiological effects and the like.

The health functional food of the present invention may be used for the purpose of preventing or treating candidiasis infection. When using the composition including lipoteichoic acid of the present invention as an additive, the composition including lipoteichoic acid may be added as is or used together with other foods or food ingredients, and it may be used appropriately according to conventional methods. Examples of foods to which the above substance can be added include meat, sausages, bread, chocolate, candy, snacks, confectionery, pizza, ramen, other noodles, gum, dairy products including ice cream, various soups, beverages, tea, drink preparations, alcoholic beverages and vitamin complexes, and they include all health foods in the conventional sense.

In the present invention, the mixing amount of the active ingredients may be appropriately determined depending on the purpose of use (prevention, health or therapeutic treatment). Generally, when producing food or beverages, the composition including lipoteichoic acid of the present invention is added in an amount of 1 to 20% by weight, and preferably, 5 to 10% by weight, based on the raw materials. However, in the case of long-term intake for the purpose of health and hygiene or health control, the amount may be below the above range, and since there is no problem in terms of safety, the active ingredient may be used in an amount higher than the above range. There are no special restrictions on the types of foods above. The beverage composition of the present invention may contain various flavoring agents or natural carbohydrates as additional ingredients like conventional beverages. The above-mentioned natural carbohydrates include monosaccharides such as glucose and fructose, disaccharides such as maltose and sucrose, polysaccharides such as dextrin and cyclodextrin, and sugar alcohols such as xylitol, sorbitol and erythritol. As a sweetener, natural sweeteners such as thaumatin and stevia extract or synthetic sweeteners such as saccharin and aspartame may be used. The ratio of the natural carbohydrate is generally about 0.01 to 0.04 g, and preferably, about 0.02 to 0.03 g, per 100 mL of the composition of the present invention. In addition, the composition of the present invention may contain various nutrients, vitamins, electrolytes, flavors, colorants, pectic acid and salts thereof, alginic acid and salts thereof, organic acids, protective colloidal thickeners, pH adjusters, stabilizers, preservatives, glycerin, alcohol, carbonating agents used in carbonated drinks and the like. In addition, the composition of the present invention may contain pulp for the production of natural fruit juice, fruit juice drinks and vegetable drinks. These ingredients may be used independently or in combination. The ratio of these additives is not very important, but is generally selected in the range of 0.01 to 0.1 parts by weight based on 100 parts by weight of the composition of the present invention.

Additionally, the present invention provides a livestock feed additive, including the composition.

When used as a feed additive in the present invention, the composition may be a highly concentrated solution of 20 to 90% or may be prepared in powder or granule form. The feed additives may include any one or at least one from organic acids such as citric acid, malic acid, adipic acid, lactic acid and malic acid, phosphates such as sodium phosphate, potassium phosphate, acid pyrophosphate and polyphosphate (polymerized phosphate), and natural antioxidants such as polyphenol, catechin, alpha-tocopherol, rosemary extract, vitamin C, green tea extract, licorice extract, chitosan, tannic acid, phytic acid and the like. When used as a feed additive, the composition may be formulated in the form of a normal feed and may include common feed ingredients.

In the present invention, the feed additive and feed may further include grains such as ground or pulverized wheat, oats, barley, corn and rice; vegetable protein feeds, such as those based on rape, soybean and sunflower; animal protein feeds such as blood meal, meat meal, bone meal and fish meal; dry ingredients consisting of sugar and dairy products, such as various powdered milk and whey powder and the like, and in addition to the above, they may further include nutritional supplements, digestion and absorption enhancers, growth promoters and the like.

In the present invention, the feed additive may be administered to animals alone or in combination with other feed additives in an edible carrier. Additionally, the feed additive may be easily administered to animals as top dressing, by mixing the same directly into animal feed, or in an oral dosage form separate from the feed. When the feed additive is administered separately from animal feed, it may be prepared into an immediate-release or sustained-release formulation by combining the same with a pharmaceutically acceptable edible carrier, as is well known in the art. These edible carriers may be solid or liquid, such as corn starch, lactose, sucrose, soy flakes, peanut oil, olive oil, sesame oil and propylene glycol. When a solid carrier is used, the feed additive may be a tablet, capsule, powder, troche or sugar-containing tablet or a top dressing in micro-disperse form. When a liquid carrier is used, the feed additive may be in the form of gelatin soft capsules, syrup, suspension, emulsion or solution.

In addition, the feed additive and feed may contain auxiliaries such as preservatives, stabilizers, wetting or emulsifying agents, solution accelerators and the like. The feed additive may be used by adding the same to animal feed by injecting, spraying or mixing.

The feed or feed additive in the present invention may be applied to a number of animal diets, including mammals, poultry and fish.

In the present invention, animals include mammals, and as the mammals, they may be used for pigs, cattle, sheep, goats and laboratory rodents, and in addition to the laboratory rodents, it may be used for pets (e.g., dogs, cats), and as the poultry, it may also be used for chicken, turkey, duck, goose, pheasant and quail, and as the fish, it may be used for trout, but the present invention is not limited thereto.

By including the composition of the present invention, it may be used as pet snacks, oral care products, hygiene products and health functional foods for animals, and it may be used as any pet product without limitation to the above-mentioned examples.

Additionally, the present invention provides a foodstuff wash, including the composition.

### [Advantageous Effects]

The antifungal composition including lipoteichoic acid according to the present invention exhibits the effects of inhibiting biofilm formation and inhibiting fungal infection by reducing the adhesion of *Candida* fungi, and is highly safe in the human body through the use of biocompatible materials, and as an antifungal agent that can reduce side effects, it can not only effectively treat candidiasis, which is an infectious disease caused by the *Candida* fungus, but also control *Candida* infections through medical devices that are inserted into the human body.

### [Description of Drawings]

FIG. 1 shows the results confirming the effect of reducing the surface adhesion of *Candida albicans* by LreLTA.
FIG. 2 shows the results confirming the effect of reducing the surface adhesion of *Candida albicans* that had already attached and formed colonies by LreLTA.
FIG. 3 shows the results confirming the effect of reducing the surface adhesion of *Candida albicans* by LTA isolated and purified from various bacteria.
FIG. 4 shows the results confirming the effect of reducing the surface adhesion of *Candida albicans* by treatment with heat shock protein 70 inhibitor and LreLTA.
FIG. 5 shows the results confirming the effect of reducing the surface adhesion of *Candida albicans* when treated with LreLTA using an *ex vivo* model.
FIG. 6 shows the results confirming the effect of reducing the surface adhesion of *Candida albicans* when treated with LreLTA using an *in vivo* model.

### [Modes of the Invention]

Hereinafter, the constitutions and effects of the present invention will be described in more detail through examples. These examples are only for illustrating the present invention, and the scope of the present invention is not limited by these examples.

### Example 1: Isolation and purification of lipoteichoic acid from Lactobacillus reuteri

In order to purify lipoteichoic acid from *L. reuteri, L. reuteri* ATCC 23272 strain was used. *L. reuteri* was inoculated into the de man, rogosa and sharpe broth (MRS broth) and cultured at 37°C for 24 hours, and then, the bacteria were harvested by centrifugation. The harvested bacteria were homogenized by using an ultrasonicator, the homogenate was dissolved in butanol, which is an organic solvent, and the lipid layer was separated by centrifugation. Afterwards, the separated extract was purified by sequentially using hydrophobic interaction chromatography and anion exchange chromatography, and the purified product was freeze-dried to obtain the final dried product.

### Example 2: Effect of L. reuteri LTA (LreLTA) on reducing surface adhesion of Candida albicans

In order to determine the effect of LreLTA on the surface adhesion of C. *albicans,* the wild-type strain SC5314 was used. After culturing *C. albicans* in Yeast Extract-Peptone-Dextrose (YPD) medium, *C. albicans* was prepared at 1 × 10⁴ CFU/ml and treated with LreLTA at concentrations of 3, 10 and 30 µg/mL. After culturing at 37°C for 24 hours, the degree of adhesion to the biofilm surface was compared through 0.1% crystal violet staining. As a result of the experiment, as shown in FIG. 1A, it was confirmed that LreLTA weakens the surface adhesion *of C. albicans* (FIG. 1A).

Additionally, in order to confirm whether LreLTA exhibits the same effect on clinical isolates in addition to the wild-type strain, *C. albicans* KCOM 3253, KCOM 3258, NCCP 32701, NCCP 32702 and NCCP 32703 strains were cultured in YPD medium, and *C. albicans* was prepared at 1 × 10⁷ CFU/mL and treated with LreLTA at a single concentration of 30 µg/mL. After culturing at 37°C for 24 hours, the degree of maintenance of biofilm surface adhesion was compared through 0.1% crystal violet staining. As a result of the experiment, it was confirmed that LreLTA also weakens the surface adhesion of clinical isolates of *C*. *albicans* (FIGS. 1B to F).

### Example 3: Effect of LreLTA on pre-attached C. albicans clusters

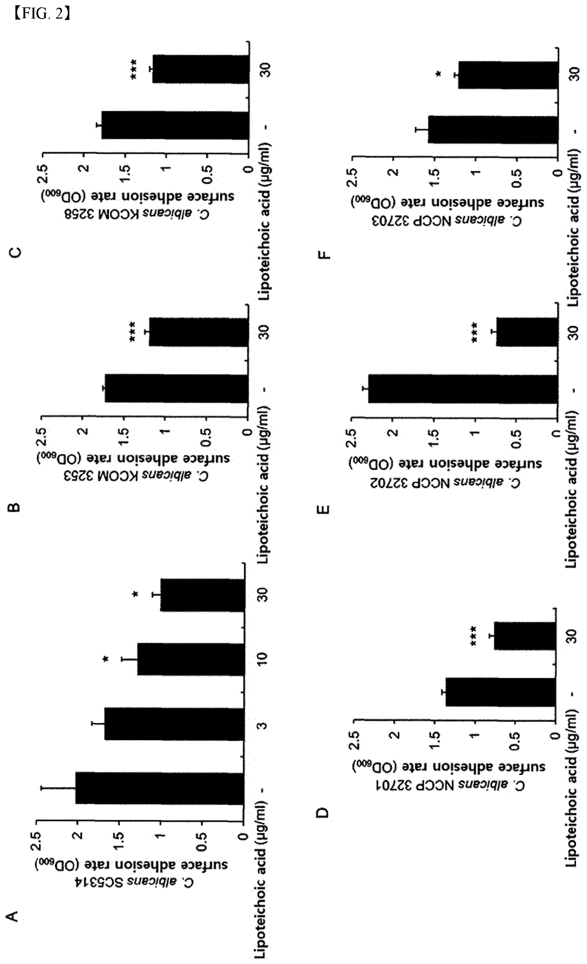

In order to confirm whether LreLTA has the same effect of weakening the adhesion *of C. albicans* in the pre-formed *C. albicans* biofilm, *C. albicans* SC5314 strain was cultured in YPD medium and prepared at 1 × 10⁴ CFU/mL to form biofilm at 37°C for 24 hours. Afterwards, the formed biofilm was treated with LreLTA at different concentrations of 3, 10 and 30 µg/mL and cultured at 37°C for 24 hours, and the degree of maintenance of adhesion to the biofilm surface was compared by 0.1% crystal violet staining. As a result of the experiment, it was confirmed that LreLTA shows the effect of reducing surface adhesion even in pre-formed biofilms of *C. albicans* (FIG. 2A).

Additionally, in order to confirm whether LreLTA shows the same effect on clinical isolates, *C. albicans* KCOM 3253, KCOM 3258, NCCP 32701, NCCP 32702 and NCCP 32703 strains were cultured in YPD medium and prepared at 1 × 10⁷ CFU/mL, and biofilm was formed at 37°C for 24 hours. Afterwards, the pre-formed biofilm was treated with LreLTA at a single concentration of 30 µg/mL and cultured at 37°C for 24 hours, and the degree of maintenance of adhesion to the biofilm surface was compared by 0.1% crystal violet staining. As a result of the experiment, it was confirmed that LreLTA shows the same effect on clinical isolates (FIGS. 2B to F).

### Example 4: Effect of isolated and purified LTA from different Lactobacillus species and Gram-positive bacteria on decreasing C. albicans surface adhesion

In order to determine the effect of LTA isolated and purified from various other lactic acid bacteria and Gram-positive bacteria on the surface adhesion of *C*. *albicans,* LTA that was isolated and purified from the, 5 types of lactic acid bacteria and 3 types of Gram-positive bacteria were used. *C. albicans* SC5314 strain was cultured in YPD medium, the cell number was prepared at 1 × 10⁴ CFU/mL, and 8 types of LTA were treated at a single concentration of 30 µg/mL. After culturing at 37°C for 24 hours, the degree of adhesion to the biofilm surface was compared by 0.1% crystal violet staining.

As a result of the experiment, as shown in FIG. 3, it was confirmed that all of 7 types of LTA except LaLTA weakened the surface adhesion of *C. albicans,* and among the LTAs isolated from probiotics, which are beneficial bacteria for the human body, particularly, LreLTA effectively weakened the surface adhesion of *C. albicans* (LaLTA: *Lactobacillus acidophilus* lipoteichoic acid; LrLTA: *Lactobacillus rhamnosus GG* lipoteichoic acid; *LpLTA: Lactobacillus plantarum* lipoteichoic acid; LcLTA: *Lactobacillus casei* lipoteichoic acid; LreLTA: *Lactobacillus reuteri* lipoteichoic acid; EfLTA: *Enterococcus fecalis* lipoteichoic acid; SmLTA: *Streptococcus mutans* lipoteichoic acid; BsLTA: *Bacillus subtilis* lipoteichoic acid).

### Example 5: Verification of efficacy of LreLTA using heat shock protein 70 inhibitor VER-155008

According to previous research, Hsp70 is known to play a role in controlling overall virulence factors and drug resistance in *C. albicans,* and thus, the inventors of the present invention conducted experiments by utilizing VER-155008, which is an Hsp70 inhibitor.

In order to determine the effect of VER-155008 on reducing *C. albicans* surface adhesion by LreLTA, *C. albicans* SC5314 strain was cultured in YPD medium and prepared at 1 × 10⁴ CFU/mL, and LreLTA at a single concentration of 30 µg/mL and VER-155008 at concentrations of 1, 10, 50 and 100 µM were simultaneously treated. After culturing for 24 hours, the degree of adhesion maintenance to the surface was compared by 0.1% crystal violet staining.

As a result of the experiments, as shown in FIG. 4, it was confirmed that the efficacy of reducing the surface adhesion of LreLTA was decreased as VER-155008 was treated at high concentrations, the effectiveness of reducing surface adhesion by LreLTA decreased, and consequently, it was confirmed that lipoteichoic acid interacts with Hsp70 of *C. albicans* to weaken adhesion and cohesion.

### Example 6: Verification of the inhibitory effect of LreLTA on C. albicans adhesion through ex vivo model

To determine whether the surface adhesion-inhibiting effect of LreLTA on *C. albicans* also occurs in the biological tissue, we used the *C. albicans* SC5314 strain to assess colony-forming units (CFU). Wild-type mice of the C57BL/6 strain were purchased from Dooyeol Biotech and used as experimental animals for the *ex vivo* model, and animal experiments were conducted in strict compliance with the ethical regulations for laboratory animal research. After the mouse was euthanized, the tongue was extracted. The excised tongues were co-cultured with *C. albicans* SC5314 at a concentration of 1 × 10⁷ CFU/ml in YPD medium, along with a simultaneous treatment of LreLTA at a single concentration of 30 µg/ml. After co-culturing at 37°C for 1 hour, the samples were transferred to fresh medium and incubated at 37°C for 24 hours. Afterwards, tongue tissue cultured with the fungus was homogenized by using a homogenizer and then inoculated onto agar medium to compare the degree of colony forming units.

Consistent with the observations in Figure 5, the tongue tissues treated with LreLTA exhibited a statistically significant reduction in CFU values of *C. albicans* compared to the control group treated with water. This confirms that LreLTA can attenuate the adhesion of *C*. *albicans* in biological tissues.

### Example 7: Verification of the inhibitory effect of LreLTA on C. albicans adhesion through in vivo model

A mouse oral candidiasis model was used to confirm whether the effect of LreLTA on weakening the surface adhesion of *C. albicans* shows the same phenomenon in animal disease models. Wild-type mice of the C57BL/6 strain were purchased from Dooyeol Biotech and used as experimental animals for the *in vivo* model, and animal experiments were performed in strict compliance with the ethical regulations for laboratory animal research. A cotton swab containing *C. albicans* SC5314 at a concentration of 1 × 10⁷ CFU/mL cultured in YPD medium was inserted into the sublingual area of mouse oral cavity to induce infection, followed by simultaneous treatment with LreLTA at a single concentration of 50 µg/mL. Afterwards, water for injection (control group) or LreLTA (experimental group) was administered to the oral cavity of mice every day. After developing candidiasis for 5 days, the mouse was euthanized, the tongue tissue was taken, and it was homogenized by using a homogenizer, and then, it was inoculated onto Agar medium to compare the degree of colony forming units.

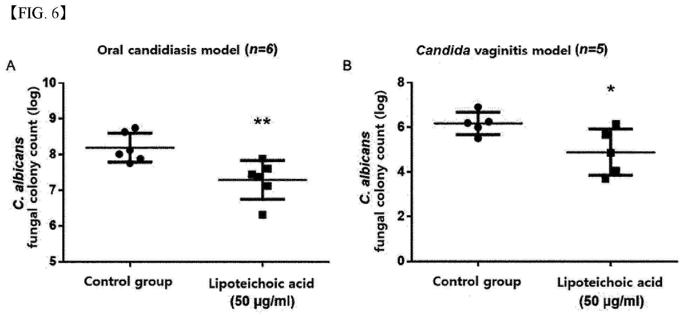

As a result of the experiments, the CFU value of *C*. *albicans* was statistically significantly reduced in the tongue tissue of mice treated with LreLTA compared to the tongue tissue treated with the water for injection which was the control group, and through this, it was confirmed that LreLTA can effectively inhibit *C. albicans* infection in a mouse oral candidiasis disease model (FIG. 6A).

Additionally, in order to confirm whether LreLTA shows the same effect in the mouse *Candida* vaginitis model, *C. albicans* SC5314 strain was cultured in YPD medium, and the number of cells was prepared at 1 × 10⁷ CFU/mL and injected into the vagina of the mouse, and then, LreLTA was simultaneously treated at a single concentration of 50 µg/mL. Afterwards, water for injection (control group) or LreLTA (experimental group) was injected into the vagina of mice every day. After developing *Candida* vaginitis for 7 days, the mouse was euthanized, the vagina tissue was removed, and it was homogenized by using a homogenizer, and then, it was inoculated onto Agar medium to compare the degree of colony forming units.

As a result of the experiments, the CFU value of *C. albicans* was statistically significantly reduced in the vaginal tissue of mice treated with LreLTA compared to the vaginal tissue treated with water for injection, which was the control group, and through this, it was confirmed that LreLTA can weaken the adhesion of *C. albicans* and effectively inhibit infection in a mouse *Candida* vaginitis disease model (FIG. 6B).

## Claims

1. *A Candida* antifungal composition, comprising:
i) lipoteichoic acid or a pharmaceutically acceptable salt thereof; or
ii) a cell wall extract comprising lipoteichoic acid.

2. The *Candida* antifungal composition of claim 1, wherein the lipoteichoic acid or cell wall extract comprising lipoteichoic acid is derived from Gram-positive bacteria.

3. The *Candida* antifungal composition of claim 1, wherein the lipoteichoic acid or the cell wall extract comprising lipoteichoic acid is derived from any one selected from the group consisting of *Lactobacillus reuteri, Lactobacillus acidophilus, Lactobacillus rhamnosus* GG, *Lactobacillus plantarum, Lactobacillus casei, Lactobacillus paracasei, Lactobacillus fermentum, Lactobacillus delbrueckii subsp. bulgaricus, Lactobacillus helveticus, Lactobacillus fermentum, Lactobacillus salilvarius, Lactococcus lactis, Enterococcus faecalis, Enterococcus faecium, Streptococcus thermophilus, Bifidobacterium bifidum, Bifidobacterium breve, Bifidobacterium longum, Bifidobacterium animalis subsp. Lactis* and *Bacillus subtilis.*

4. The *Candida* antifungal composition of claim 1, wherein the cell wall extract is extracted by using water, C₁ to C₄ lower alcohol or a mixture thereof as a solvent.

5. The *Candida* antifungal composition of claim 1, wherein the *Candida* is a fungus selected from the group consisting of *Candida albicans, Candida tropicalis, Candida pseudotropicalis, Candida krusei, Candida glabrata* and a mixture thereof.

6. The *Candida* antifungal composition of claim 1, wherein the Candida antifungal composition reduces the surface adhesion of *Candida* fungi.

7. A pharmaceutical composition for preventing or treating candidiasis infection, comprising the *Candida* antifungal composition according to any one of claims 1 to 6.

8. The pharmaceutical composition of claim 7, wherein the candidiasis infection is at least any one selected from the group consisting of vulvitis, vaginitis, esophagitis, paronychia, oropharyngitis, digestive candidiasis, mucosal candidiasis, cutaneous candidiasis, *Candida* fungemia, *Candida* endophthalmitis, *Candida* peritonitis, *Candida* osteoarthritis, *Candida* spondylitis and hepatosplenic candidiasis.

9. A coating agent, comprising the *Candida* antifungal composition according to any one of claims 1 to 6.

10. The coating agent of claim 9, wherein the coating agent is used in medical devices, medical materials, medical implants, building interior materials, household goods, industrial products, electronic products, clothing and artificial organs.

11. A health functional food, comprising the *Candida* antifungal composition according to any one of claims 1 to 6.

12. A livestock feed additive, comprising the *Candida* antifungal composition according to any one of claims 1 to 6.

13. A foodstuff wash, comprising the *Candida* antifungal composition according to any one of claims 1 to 6.
